# EUROPEAN PATENT APPLICATION

(11) **EP 4 512 374 A1**
(43) Date of publication of application: **26.02.2025**
(21) Application number: 24195675.4
(22) Date of filing: 21.08.2024
(51) Int. Cl.: A61F 2/36, A61F 2/46, A61F 2/30

(54) **EXPANDABLE TRIAL FEMORAL NECK**

(30) Priority: 23.08.2023 US 202318454472
(71) Applicant: Globus Medical, Inc., Audubon, PA 19403 (US)
(72) Inventor: LOIACONO, John, COLLEGEVILLE, 19426 (US); STUMPO, David, TRAPPE, 19426 (US); MCGONAGLE, Carly, PHILADELPHIA, 19128 (US); CAPOZZOLI, Joseph, MT. LAUREL, 08054 (US); VINSHTOK, Yevgeniy, DOWNINGTOWN, 19335 (US)
(74) Representative: Morabito, Sara

(57) **Abstract**

A trial femoral assembly (10) for hip arthroplasty is disclosed that includes a head, a neck (12) connected to the head, and a stem (22) or broach connected to the neck, wherein the neck is expandable and collapsible. Also, the stem or broach can move laterally in relation to the longitudinal axis (16) of the neck. A first embodiment includes a notched rod (60) located within a housing (54) and within a circular lower neck portion having a tilted spiral cam (52) that interacts with the notched rod to provide movement involving extension or retraction of the neck. A second embodiment includes a bevel gear (80) located on an outer surface of the head that engages a screw (92) located inside the head to move the neck to provide extension or retraction. A third embodiment includes at least three nested, telescoping threaded elements (102, 104, 106) with oppositely handed threads that extend or retract the neck.

## Description

### TECHNICAL FIELD

The present disclosure generally relates to an expandible trial femoral neck that allows the surgeon to determine appropriate trial implants (with either a head offset or a stem offset) without dislocating the patient's joint.

### BACKGROUND

In total hip arthroplasty, after broaching and/or reaming the femoral canal and reaming the acetabulum, the surgeon will place different trial components and reduce the joint to find the right range of motion, soft tissue balance, and leg length. The trial components include trial femoral heads and trial femoral necks, and a surgeon will often times trial multiple different offset options to find the best fit for the patient.

Trial heads are offered in sizes 28 millimeters, 32 millimeters, and 36 millimeters. The 28-millimeter heads are offered in offsets of-5 millimeters, -3.5 millimeters, 0 millimeters, +3.5 millimeters, +7 millimeters, +10.5 millimeters, and +12 millimeters. The 32 millimeters and 36 millimeters trial heads are offered in -3.5 millimeters, 0 millimeters, +3.5 millimeters, +7 millimeters, and +10.5 millimeters.

The trial necks are offered in three sizes for use with stems 1/2/3/4, 5/6/7/8, and 9/10/11/12. In addition, standard and lateralized options are available for each size for a total of six trial necks.

Therefore, the total SKUs for just trial heads and trial necks adds up to twenty-three SKUs, taking up a large portion of the graphics cases utilized in surgery. This large number of SKUs typically involves the trial heads being marked or color coded and requires significant space on the instrument tray. This situation also leads to confusion for the scrub technician and surgeon. Thus, there exists a need in the art for an adjustable/expandable trial neck. The trial neck could adjust from standard to lateralized, and/or could expand over a range of trial head sizes.

### SUMMARY

The following objects, features, advantages, aspects, and/or embodiments are not exhaustive and do not limit the overall disclosure. No single embodiment need provide each and every object, feature, or advantage. Any of the objects, features, advantages, aspects, and/or embodiments disclosed herein can be integrated with one another, either in full or in part.

It is a primary object, feature, and/or advantage of the present disclosure to improve on or overcome the deficiencies in the art.

An aspect of the present disclosure is a trial femoral assembly for hip arthroplasty, which includes a head, a neck connected to the head, and a stem or broach connected to the neck, wherein the neck is expandible and collapsible.

Another aspect of the present disclosure is a stem or broach that can move laterally in relation to the longitudinal axis of the neck.

Yet another aspect of the present disclosure is a notched rod located within a housing and within a circular lower neck portion having a tilted spiral cam that interacts with the notched rod to provide movement involving extension or retraction of the neck.

Another feature of the present disclosure is a tilted spiral cam includes a protrusion that engages an opening in the housing to provide a securing mechanism.

Yet another aspect of the present disclosure is a bevel gear located on an outer surface of the head that engages a screw located inside the head to move the neck to provide extension or retraction.

Still, yet another feature of the present disclosure is a driver tool attached to a bevel pinion gear that rotates the bevel gear when the bevel pinion gear is in contact with the bevel gear.

Another feature of the present disclosure is a coupling sleeve having a flange member at an outer end that engages a slot located internally within the head.

Still, another aspect of the present disclosure is a coupling sleeve attached to the neck.

A further feature of the disclosure is a plurality of nested, telescoping threaded elements with oppositely handed threads that extend or retract the neck.

Still, another feature of the present disclosure is a first nested, telescoping threaded element attached to the stem or broach, a third nested, telescoping threaded element attached to the head, and a second nested, telescoping threaded element located between the first nested, telescoping threaded element attached to the stem or broach, and the third nested, telescoping threaded element attached to the head.

Still, yet another feature of the present disclosure is a threaded nut that can travel up and down the first nested, telescoping threaded element and an anti-rotation guide located between the first nested, telescoping threaded element and the third nested, telescoping threaded element to lock rotation of the first nested, telescoping threaded element and the third nested, telescoping threaded element in relationship to each other.

Another aspect of the present disclosure is there is at least a fifty percent overlap between the plurality of nested, telescoping threaded elements at any one time, and the threaded nut has threads in the same direction as the third nested, telescoping threaded element and of an opposite direction to that of the first nested, telescoping threaded element, wherein the third nested, telescoping threaded element is not rotating relative to the first nested, telescoping threaded element so that rotation of the head does not affect an overall length of the trial femoral assembly.

An additional feature of the disclosure is a method of placing a trial femoral assembly on a patient that includes a head, a neck connected to the head, and a stem or broach connected to the neck, where the surgeon can expand or contract the neck to reduce the patient's joint and find the preferred range of motion, soft tissue balance and leg length for the patient.

It is still yet a further object, feature, and/or advantage of the present disclosure is a method of laterally moving the stem or broach in relation to a longitudinal axis of the neck.

Yet another feature of the method of the present disclosure is utilizing a notched rod located within a housing and within a circular lower neck portion having a tilted spiral cam that interacts with the notched rod to provide movement of the neck for extension or retraction.

It is still yet another feature of the method of the present disclosure is utilizing a bevel gear located on the outer surface of the head that engages a screw located inside the head to provide extension or retraction of the neck.

Another feature of the method of the present disclosure is utilizing a driver tool attached to the bevel pinion gear that rotates the bevel gear when the bevel pinion gear is in contact with the bevel gear.

It is yet another feature of the method of the present disclosure is utilizing a plurality of nested, telescoping threaded elements with oppositely handed threads to extend or retract the neck.

It is a further object, feature, and/or advantage of the method of the present disclosure involves utilizing a first nested, telescoping threaded element attached to the stem or broach, a third nested, telescoping threaded element attached to the head, and a second nested, telescoping threaded element located between the first nested, telescoping threaded element and the third nested, utilizing a first nested, telescoping threaded element attached to the stem or broach, a third nested, telescoping threaded element attached to the head, and a second nested, telescoping threaded element located between the first nested, telescoping threaded element and the third nested,

It is still yet a further object, feature, and/or advantage of the method of the present disclosure involving utilizing a threaded nut that can travel up and down the first nested, telescoping threaded element and an anti-rotation guide located between the first nested, telescoping threaded element and the third nested, telescoping threaded element to lock rotation of the first nested, telescoping threaded element and the third nested, telescoping threaded element in relationship to each other.

These and/or other objects, features, advantages, aspects, and/or embodiments will become apparent to those skilled in the art after reviewing the following brief and detailed descriptions of the drawings. The present disclosure encompasses (a) combinations of disclosed aspects and/or embodiments and/or (b) reasonable modifications not shown or described.

### BRIEF DESCRIPTION OF THE DRAWINGS

Several embodiments in which the present disclosure can be practiced are illustrated and described in detail, wherein like reference characters represent like components throughout the several views. The drawings are presented for exemplary purposes and may not be to scale unless otherwise indicated.

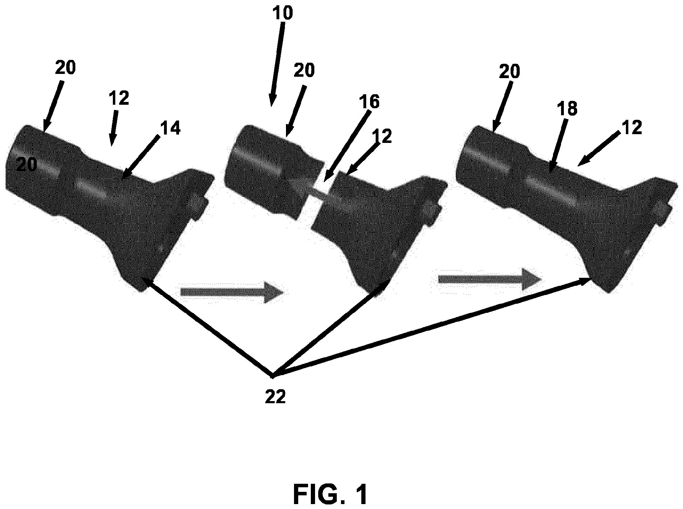

**FIG. 1** is a perspective view of a trial femoral neck from left to right in a first image of a contracted neck position, a second image showing a potential neck expansion, and a third image of an expanded neck position.

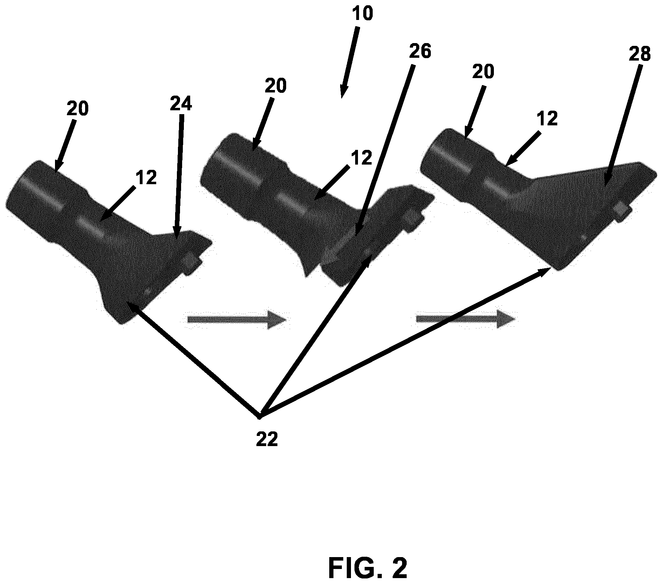

**FIG. 2** is a perspective view of a trial femoral neck from left to right in a first image of a standard position of the stem in relation to the neck, a second image showing the potential lateral offset of the stem, and a third image of a laterally shifted stem in relation to the neck.

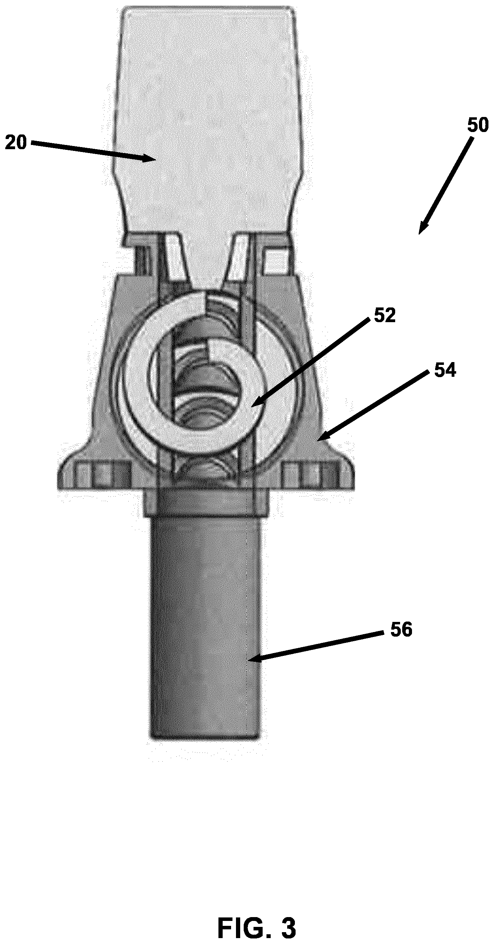

**FIG. 3** is a front view of a first embodiment of a neck expansion and contraction mechanism trial femoral neck with a cutaway view within a housing that includes a tilted spiral cam.

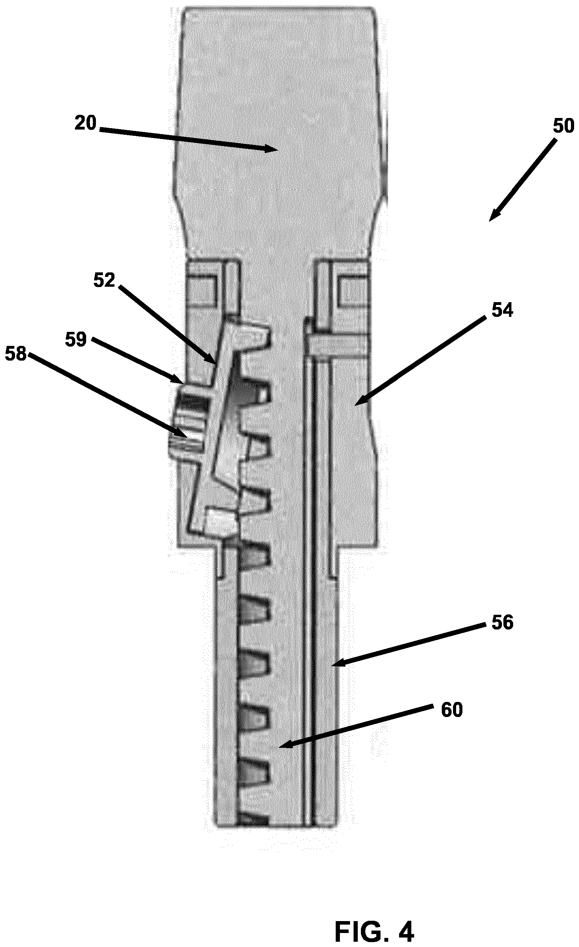

**FIG. 4** is a side view of a first embodiment of a neck expansion and contraction mechanism trial femoral neck with a cutaway view of a notched rod interacting with the tilted spiral cam shown in **FIG. 3****.**

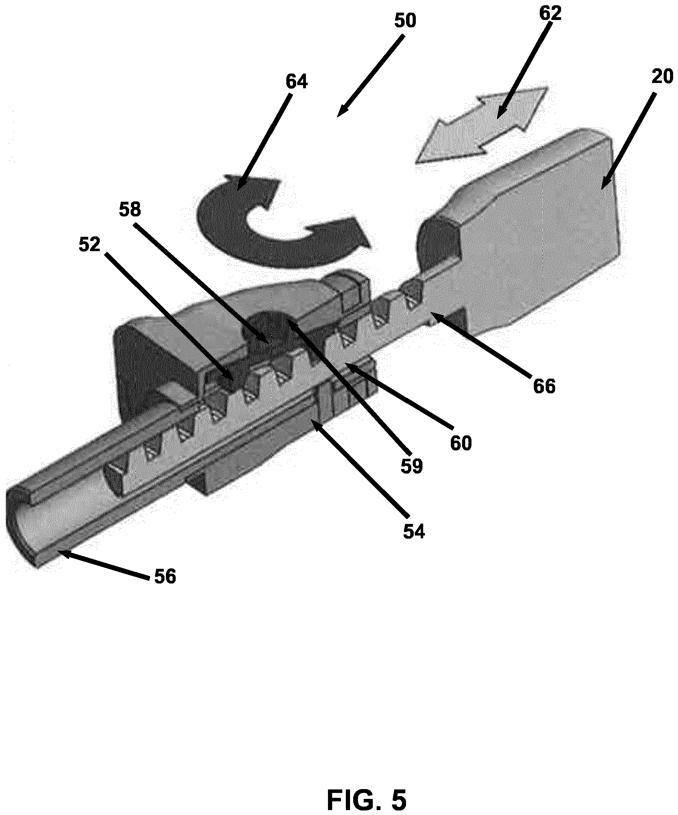

**FIG. 5** is a side view of a first embodiment of a neck expansion and contraction mechanism trial femoral neck rotated ninety degrees from **FIG. 4** with a cutaway view of a notched rod interacting with the tilted spiral cam with the notched rod extended and attached to the head and the bottom of the notched rod secured in a circular lower neck enclosure.

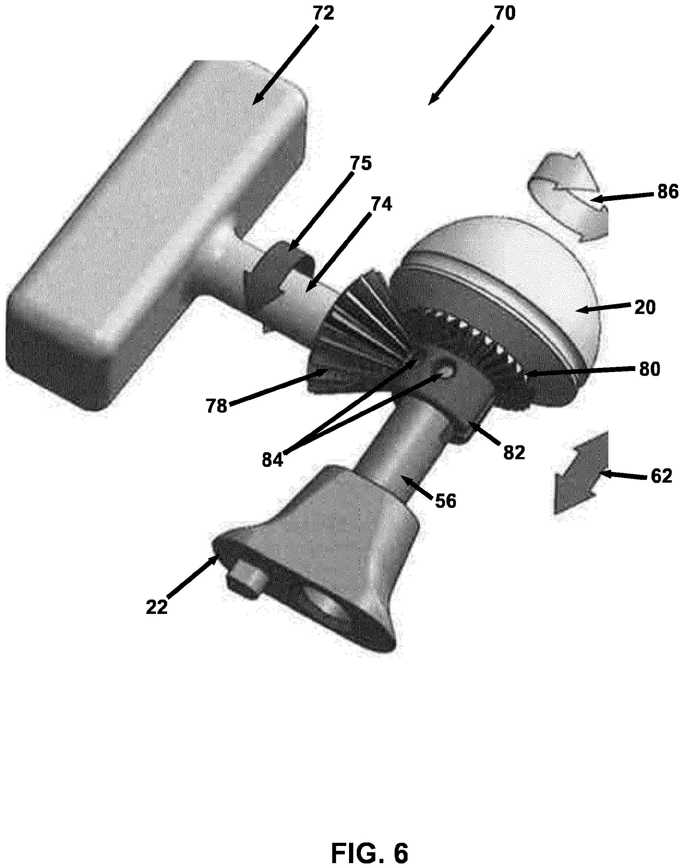

**FIG. 6** is a perspective view of a second embodiment for a neck expansion and contraction mechanism trial femoral neck that includes a driver tool that rotates a bevel pinion gear that engages a bevel gear on a head that expands or contracts the length of the neck.

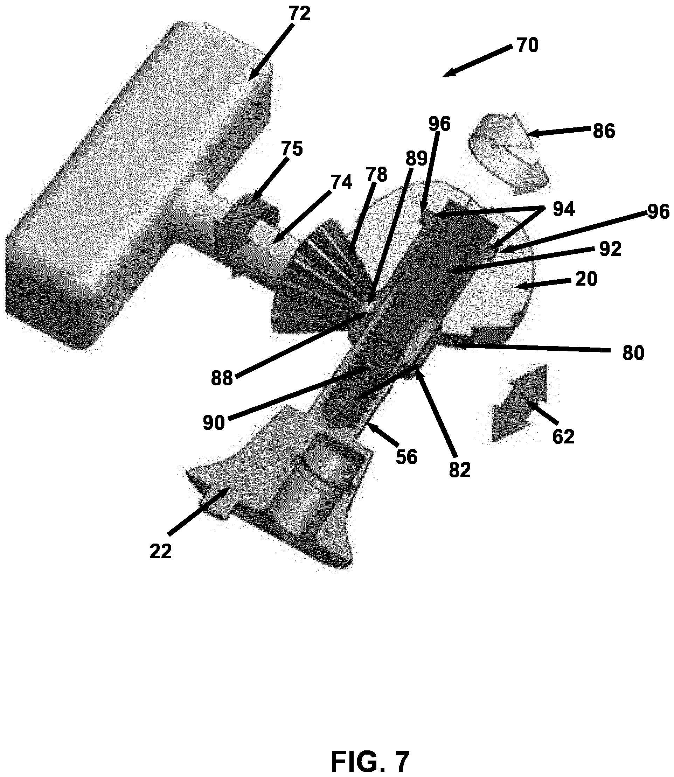

**FIG. 7** is a perspective cutaway view of a second embodiment for a neck expansion and contraction mechanism trial femoral neck that includes a driver tool that rotates a bevel pinion gear that engages a bevel gear on a head that expands or contracts a length of a neck by engaging a screw located within a coupling sleeve that is secured within a head.

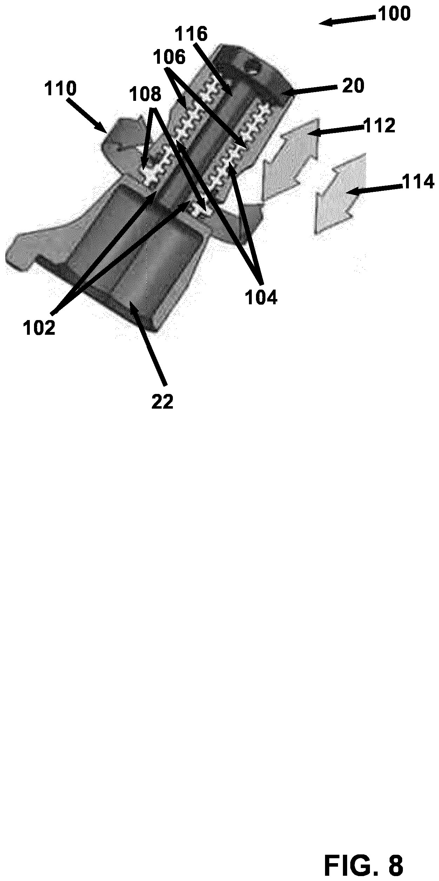

**FIG. 8** is a cutaway perspective view of a third embodiment for a neck expansion and contraction mechanism trial femoral neck that includes a first nested, telescoping threaded element attached to the stem, a third nested, telescoping threaded element attached to the head, and a second nested, telescoping threaded element located between the first nested, telescoping threaded element and the third nested, telescoping threaded element with a threaded nut that can travel up and down the second nested, telescoping threaded element and an anti-rotation guide located between the second nested, telescoping threaded element and the third nested, telescoping threaded element to lock rotation of the second nested, telescoping threaded element and the third nested, telescoping threaded element in relationship to each other in a contracted position.

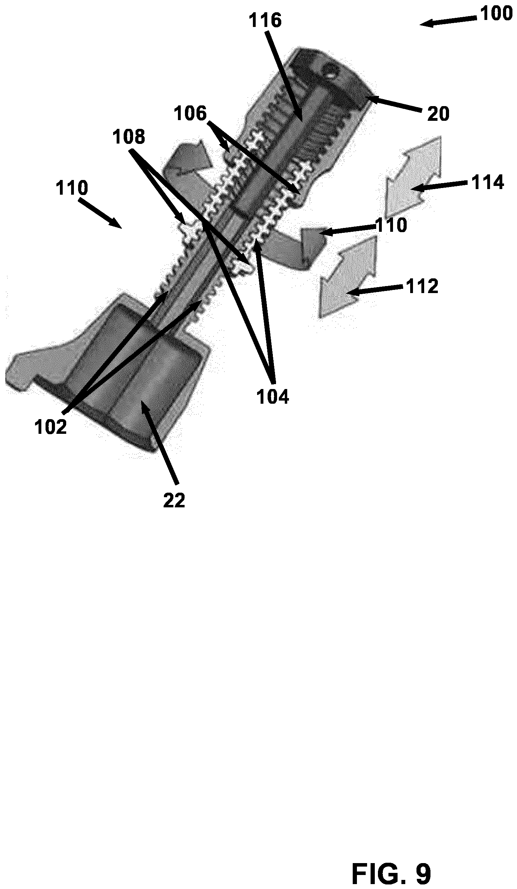

**FIG. 9** is a cutaway perspective view of a third embodiment for a neck expansion and contraction mechanism trial femoral neck shown in **FIG. 8** that includes a first nested, telescoping threaded element attached to the stem; a third nested, telescoping threaded element attached to the head, and a second nested, telescoping threaded element located between the first nested, telescoping threaded element and the third nested, telescoping threaded element with a threaded nut that can travel up and down the second nested, telescoping threaded element and an anti-rotation guide located between the second nested, telescoping threaded element and the third nested, telescoping threaded element to lock rotation of the second nested, telescoping threaded element and the third nested, telescoping threaded element in relationship to each other in an expanded position.

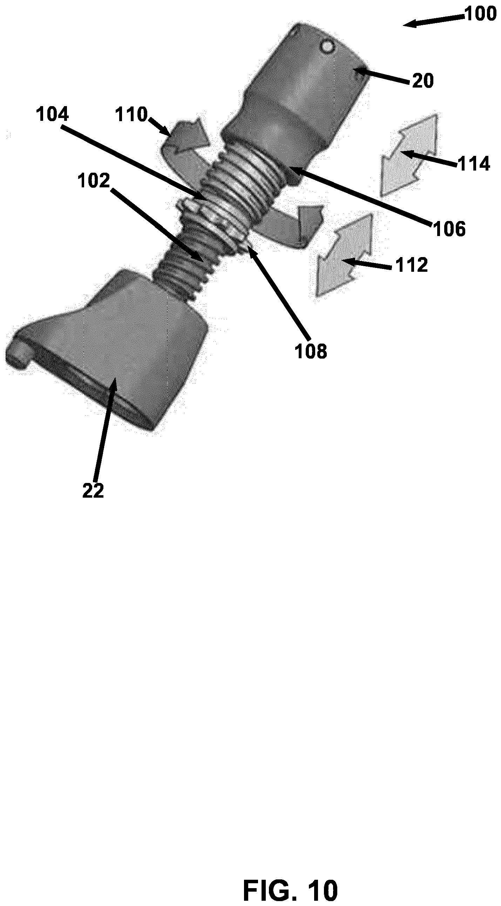

**FIG. 10** is a perspective view of a third embodiment for a neck expansion and contraction mechanism trial femoral neck shown in **FIG. 8** that includes a first nested, telescoping threaded element attached to the stem; a third nested, telescoping threaded element attached to the head, and a second nested, telescoping threaded element located between the first nested, telescoping threaded element and the third nested, telescoping threaded element with a threaded nut that can travel up and down the second nested, telescoping threaded element and an anti-rotation guide located between the second nested, telescoping threaded element and the third nested, telescoping threaded element to lock rotation of the second nested, telescoping threaded element and the third nested, telescoping threaded element in relationship to each other in an expanded position.

An artisan of ordinary skill in the art need not view, within isolated figure(s), the near-infinite distinct combinations of features described in the following detailed description to facilitate an understanding of the present disclosure.

### DETAILED DESCRIPTION

The present disclosure is not to be limited to that described herein. Mechanical, electrical, chemical, procedural, and/or other changes can be made without departing from the spirit and scope of the present disclosure. Unless otherwise indicated, no features shown or described are essential to permit basic operation of the present disclosure.

Trial femoral neck segments and trial femoral modular heads are utilized to assess proper component position, joint stability, range of motion, and leg length.

Referring now to FIG. 1, a trial femoral head and neck assembly is generally indicated by the numeral 10. The adjustable femur neck portion 12 of the trial femoral head and neck assembly 10 can adjust from standard to lateralized, and/or could expand over a range of sizes of trial trunnion 20. Moreover, the adjustable femur neck portion 12 can expand over a range of sizes that allows the surgeon to adjust the neck portion 12 while just using one neutral (+0 millimeters) trial head and would allow the surgeon to easily change the offset while the patient's joint is still reduced. The expansion mechanism of the adjustable femoral neck portion 12 will be on the internal portion of the adjustable femoral neck portion 12. It will be controlled by a separate instrument that the surgeon could turn with a click, indicating that it has adjusted to a new size. Trial trunnion 20 sizes range from -5 millimeters to+ 12 millimeters, so ideally, the trial femoral neck 20 will be able to expand throughout that entire range. There is a stem 22 for attachment to the femur. There can be a broach representative of the final stem implant geometry substituted for the stem 22. The is a first length 14 of the adjustable femur neck portion 12, a visual depiction of the expansive of neck portion along the neck axis 16 of the adjustable femur neck portion 12, and a second length 18 of the adjustable femur neck portion 12. The images in FIG. 1 show how the adjustable femoral neck portion 12 will expand along the axis of the neck to lengthen. It will expand in increments so that the user can easily tell how much they are expanding. Preferably, but not necessarily, it will also be laser marked to show the exact dimension.

Referring now to FIG. 2, another feature is to make the trial femoral head and neck 10 adjustable so that a change from standard to lateralized can be accomplished. Currently, there needs to be six total trial necks due to there being three different neck lengths, and one standard and one lateralized option for each one. If the adjustable femoral neck portion 12 can be adjusted to be lateralized, that would reduce the total number of trial necks from six to three SKUs. As shown, stem 22 can go from a standard position of the stem 24 in relation to the adjustable femoral neck portion 12, as shown by a lateral change of stem 26 in relation to the adjustable femoral neck portion 20 to a lateralized position of the stem 22 to the adjustable femoral neck portion 28. The trunnion 20 can be moved back and forth and could click into the correct place so that the user obtains tactile feedback to verify that the adjustable femoral neck portion 12 is located in the proper position. Preferably, there would be an indication showing the level of offset.

There is a first embodiment of a neck lengthening device that is generally indicated by the numeral 50 in FIGS. 3, 4, and 5. Positioned below the trunnion 20 is notched rod 60 with surfaces that connect to surfaces of a tilted spiral cam 52. The tilted spiral cam 52 is located within housing 54. This neck lengthening device 50 provides an extension of the neck portion 66, shown in FIG. 5, that cannot be back driven by pressure from the tissue or rotation of the trunnion 20. The amount of extension 66 of the trunnion 20 can be accurately controlled or precisely "dialed in" based on the dimensions of the tilted spiral cam 52. The tilted spiral cam 52 includes a protrusion 58 that engages an opening 59 in the housing 54 to provide a securing mechanism. The longitudinal neck movement is generally indicated by the numeral 62, and the circular spiral cam movement that is converted to longitudinal neck movement is indicated by the numeral 64. Housing 54 is positioned above the neck portion 56, which is cylindrical and encloses the notched rod 60.

A second embodiment of a neck lengthening device is generally indicated by the numeral 70 in FIGS. 6 and 7. There is a driver tool 72 that provides a rotational force. Numerous embodiments of various types of tools can provide this type of rotational force comparable to a hand drill. The driver tool 72 includes an enclosed rotating shaft 74 that is attached to a bevel pinion gear 78. The direction of rotation of the rotating shaft 74 is indicated by the numeral 75. The bevel pinion gear 78 engages a bevel gear 80 that is attached to the trunnion 20 with the rotation indicated by the numeral 86. As before, the neck portion 56 is positioned on top of the stem 22.

As shown in FIG. 7, there is a coupling sleeve 82 that includes a bushing 88 that retains a circular protrusion 89 associated with the bevel pinion gear 78. There is retained flange 94 as an upper component of the coupling sleeve 82 that is held by a slot 96 in the trunnion 20. The bevel gear 80 engages a screw 92, rotatable in a threaded internal passage 90, that provides the power to extend and retract the trunnion 20. The bevel gear 80 is also connected to the coupling sleeve 82, which serves to provide a proper alignment of the bevel pinion gear 78 and bevel gear 80 for engagement, as well as provide a bearing surface to guide the extension and retraction of neck portion 56 indicated by numeral 62. The coupling sleeve 82 is connected to the neck portion 56 by connecting screws 84, as shown in FIG. 6.

A third embodiment of a neck lengthening device is generally indicated by the numeral 100 in FIGS. 8, 9, and 10. This neck lengthening device 100 includes nested, telescoping, threaded elements, e.g., three, with oppositely handed threads. In an illustrative, but nonlimiting, embodiment, a first nested, telescoping threaded element 102, e.g., right-hand screw, has a thread running in one direction, e.g., clockwise, with a nut 108 with matching internal thread that can go up and down the first nested, telescoping threaded element 102.

This nut 108 has an external thread of opposite handedness, e.g., counterclockwise, which engages a third nested, telescoping threaded element 106, preferably with a matching internal thread, e.g., counterclockwise. This third nested, telescoping threaded element 106 is connected to the first nested, telescoping threaded element 102 via an anti-rotation guide 116, which locks the rotation of the first nested, telescoping threaded element 102 and third nested, telescoping threaded element 106. When the second nested, telescoping, threaded element 104 is rotated, it rides up the first nested, telescoping threaded element 102 and forces the third nested, telescoping threaded element 106 to extend in the same direction. Because all threaded elements 102, 104, and 106 are nested inside each other and are "telescoping," they maintain overlap from 50% to 100% at any one time, providing bending rigidity or this extending structure. Rotational movement is generally indicated by the numeral 110 of the threaded elements 102, 104, and 106.

Also, because the third nested, telescoping threaded element 106 is not rotating relative to the first nested, telescoping threaded element 102 that forms a base element, the rotation of the trunnion trial 20 does not affect the overall length of the neck lengthening device 100. The longitudinal extension and contraction of the second nested, telescoping, threaded element 104 is indicated by numeral 112, and the longitudinal extension and contraction of the third nested, telescoping, threaded element 106 is indicated by numeral 114.

The advantage of this trial femoral head and neck assembly 10 is an expandable trial neck is that it will reduce operating room time by allowing the surgeon to determine the appropriate trial implants (either head offset or stem offset) without dislocating the joint. The surgeon will be able to use an instrument that could provide tactile feedback by clicking at each offset size so that instead of dislocating the joint, replacing the head, and reducing the joint again, the surgeon will be able to leave the joint reduced and turn the instrument to change the offset size....

This technology will also reduce instrumentation significantly because there could be one trial neck for both standard and lateralized necks. There will also only need to be one neutral offset (+0 millimeter) head that the surgeon will trial with. Reducing instrumentation is especially important in joint arthroplasty due to the increasing presence of ambulatory surgery centers that do not have room to store large amounts of instrumentation. Having less instrumentation also makes things much easier for the scrub technician, making the whole procedure much smoother and quicker.

From the foregoing, it can be seen that the present disclosure accomplishes at least all of the stated objectives.

### LIST OF REFERENCE CHARACTERS

The following table of reference characters and descriptors are not exhaustive, nor limiting, and include reasonable equivalents. If possible, elements identified by a reference character below and/or those elements which are near ubiquitous within the art can replace or supplement any element identified by another reference character.

**Table 1: List of Reference Characters**

| | |
|---|---|
| **10** | Trial femoral head and neck assembly |
| **12** | Adjustable femoral neck portion |
| **14** | First length of an adjustable femoral neck portion |
| **16** | Expansion of neck portion along neck axis |
| **18** | Second length of an adjustable femoral neck portion |
| **20** | Trunnion |
| **22** | Stem or broach |
| **24** | Standard position of the stem or broach in relation to an adjustable femoral neck portion |
| **26** | Lateral change of stem or broach in relation to an adjustable femoral neck portion |
| **28** | Lateralized position of the stem or broach to an adjustable femoral neck portion |
| **50** | First embodiment of a neck-lengthening device |
| **52** | Tilted spiral cam |
| **54** | Housing |
| **56** | Neck portion |
| **58** | Protrusion |
| **59** | Opening |
| **60** | Notched rod |
| **62** | Longitudinal neck movement |
| **64** | Spiral cam movement that converts to longitudinal neck movement |
| **66** | Extension of the neck portion |
| **70** | Second embodiment of a neck-lengthening device |
| **72** | Driver tool |
| **74** | Enclosed rotating shaft |
| **75** | Direction of rotation of a rotating shaft |
| **78** | Bevel pinion gear |
| **80** | Bevel gear |
| **82** | Coupling sleeve |
| **84** | Connecting screws |
| **86** | Direction of rotation of the femoral head |
| **88** | Bevel pinion gear bushing |
| **89** | Circular protrusion |
| **90** | Threaded internal passage |
| **92** | Screw |
| **94** | Retained flange as an upper component of the coupling sleeve |
| **96** | Slot located within the trunnion for retaining the retained flange |
| **100** | Third embodiment of a neck-lengthening device |
| **102** | First nested, telescoping threaded element |
| **104** | Second nested, telescoping threaded element |
| **106** | Third outer nested, telescoping threaded element |
| **108** | Nut |
| **110** | Rotation of threaded elements |
| **112** | Direction upward and downward of the second nested, telescoping threaded element |
| **114** | Direction upward and downward of the third nested, telescoping threaded element |
| **116** | Anti-rotation guide |

### GLOSSARY

Unless defined otherwise, all technical and scientific terms used above have the same meaning as commonly understood by one of ordinary skill in the art to which embodiments of the present disclosure pertain.

The terms "a," "an," and "the" include both singular and plural referents.

The term "or" is synonymous with "and/or" and means any one member or combination of members of a particular list.

As used herein, the term "exemplary" refers to an example, an instance, or an illustration, and does not indicate a most preferred embodiment unless otherwise stated.

The term "about" as used herein refers to slight variations in numerical quantities with respect to any quantifiable variable. Inadvertent error can occur, for example, through use of typical measuring techniques or equipment or from differences in the manufacture, source, or purity of components.

The term "substantially" refers to a great or significant extent. "Substantially" can thus refer to a plurality, majority, and/or a supermajority of said quantifiable variables, given proper context.

The term "generally" encompasses both "about" and "substantially."

The term "configured" describes structure capable of performing a task or adopting a particular configuration. The term "configured" can be used interchangeably with other similar phrases, such as constructed, arranged, adapted, manufactured, and the like.

Terms characterizing sequential order, a position, and/or an orientation are not limiting and are only referenced according to the views presented.

The "invention" is not intended to refer to any single embodiment of the particular invention but encompass all possible embodiments as described in the specification and the claims. The "scope" of the present disclosure is defined by the appended claims, along with the full scope of equivalents to which such claims are entitled. The scope of the disclosure is further qualified as including any possible modification to any of the aspects and/or embodiments disclosed herein which would result in other embodiments, combinations, subcombinations, or the like that would be obvious to those skilled in the art.

The invention could be inter alia defined by the following examples:
1. A trial femoral assembly for hip arthroplasty, comprising: a head; a neck connected to the head; and a stem or broach connected to the neck, wherein the neck is expandible and collapsible.
2. The trial femoral assembly for hip arthroplasty according to Claim 1, wherein the stem or broach can move laterally in relation to the longitudinal axis of the neck.
3. The trial femoral assembly for hip arthroplasty according to Example 1, further comprising a notched rod located within a housing and within a circular lower neck portion having a tilted spiral cam that interacts with the notched rod to provide movement involving extension or retraction of the neck.
4. The trial femoral assembly for hip arthroplasty according to Example 3, wherein the tilted spiral cam includes a protrusion that engages an opening in the housing to provide a securing mechanism.
5. The trial femoral assembly for hip arthroplasty according to Example 1, further comprising a bevel gear located on an outer surface of the head that engages a screw located inside the head to move the neck to provide extension or retraction.
6. The trial femoral assembly for hip arthroplasty according to Example 5, further comprising a driver tool attached to a bevel pinion gear that rotates the bevel gear when the bevel pinion gear is in contact with the bevel gear.
7. The trial femoral assembly for hip arthroplasty according to Example 5, further comprising a coupling sleeve having a flange member at an outer end that engages a slot located internally within the head.
8. The trial femoral assembly for hip arthroplasty according to Example 7, wherein the coupling sleeve is attached to the neck.
9. The trial femoral assembly for hip arthroplasty according to Example 1, further comprising a plurality of nested, telescoping threaded elements with oppositely handed threads that extend or retract the neck.
10. The trial femoral assembly for hip arthroplasty according to Example 9, further comprising a first nested, telescoping threaded element attached to the stem or broach, a third nested, telescoping threaded element attached to the head, and a second nested, telescoping threaded element located between the first nested, telescoping threaded element attached to the stem or broach and the third nested, telescoping threaded element attached to the head.
11. The trial femoral assembly for hip arthroplasty according to Example 10, further comprising a threaded nut that can travel up and down the first nested, telescoping threaded element and an anti-rotation guide located between the first nested, telescoping threaded element and the third nested, telescoping threaded element to lock rotation of the first nested, telescoping threaded element and the third nested, telescoping threaded element in relationship to each other.
12. The trial femoral assembly for hip arthroplasty according to Example 11, wherein there is at least a fifty percent overlap between the plurality of nested, telescoping threaded elements at any one time and the threaded nut has threads in the same direction as the third nested, telescoping threaded element and of an opposite direction to that of the first nested, telescoping threaded element, wherein the third nested, telescoping threaded element is not rotating relative to the first nested, telescoping threaded element so that rotation of the head does not affect an overall length of the trial femoral assembly.
13. A method of utilizing a trial femoral assembly for hip arthroplasty, comprising: placing a trial femoral assembly on a patient that includes a head, a neck connected to the head, and a stem or broach connected to the neck, where the surgeon can expand or contract the neck to reduce the patient's joint and find the preferred range of motion, soft tissue balance and leg length for the patient.
14. The method of utilizing a trial femoral assembly for hip arthroplasty according to Example 13, further comprising the step of laterally moving the stem or broach in relation to a longitudinal axis of the neck.
15. The method of utilizing a trial femoral assembly for hip arthroplasty according to Example 13, further comprising utilizing a notched rod located within a housing and within a circular lower neck portion having a tilted spiral cam that interacts with the notched rod to provide movement of the neck for extension or retraction.
16. The method of utilizing a trial femoral assembly for hip arthroplasty according to Example 13, further comprising utilizing a bevel gear located on the outer surface of the head that engages a screw located inside the head to provide extension or retraction of the neck.
17. The method of utilizing a trial femoral assembly for hip arthroplasty according to Example 16, further comprising utilizing a driver tool attached to the bevel pinion gear that rotates the bevel gear when the bevel pinion gear is in contact with the bevel gear.
18. The method of utilizing a trial femoral assembly for hip arthroplasty according to Example 13, further comprising utilizing a plurality of nested, telescoping threaded elements with oppositely handed threads to extend or retract the neck.
19. The method of utilizing a trial femoral assembly for hip arthroplasty according to Example 13, further comprising utilizing a first nested, telescoping threaded element attached to the stem or broach, a third nested, telescoping threaded element attached to the head, and a second nested, telescoping threaded element located between the first nested, telescoping threaded element and the third nested, telescoping threaded element to extend or retract the neck.
20. The method of utilizing a trial femoral assembly for hip arthroplasty according to Example 19, further comprising utilizing a threaded nut that can travel up and down the first nested, telescoping threaded element and an anti-rotation guide located between the first nested, telescoping threaded element and the third nested, telescoping threaded element to lock rotation of the first nested, telescoping threaded element and the third nested, telescoping threaded element in relationship to each other.

## Claims

1. A trial femoral assembly for hip arthroplasty, comprising:
a head;
a neck connected to the head; and
a stem or broach connected to the neck, wherein the neck is expandible and collapsible.

2. The trial femoral assembly for hip arthroplasty according to Claim 1 or 2, wherein the stem or broach can move laterally in relation to the longitudinal axis of the neck.

3. The trial femoral assembly for hip arthroplasty according to any one of the preceding claims, further comprising a notched rod located within a housing and within a circular lower neck portion having a tilted spiral cam that interacts with the notched rod to provide movement involving extension or retraction of the neck.

4. The trial femoral assembly for hip arthroplasty according to Claim 3, wherein the tilted spiral cam includes a protrusion that engages an opening in the housing to provide a securing mechanism.

5. The trial femoral assembly for hip arthroplasty according to any one of the preceding claims, further comprising a bevel gear located on an outer surface of the head that engages a screw located inside the head to move the neck to provide extension or retraction.

6. The trial femoral assembly for hip arthroplasty according to Claim 5, further comprising a driver tool attached to a bevel pinion gear that rotates the bevel gear when the bevel pinion gear is in contact with the bevel gear.

7. The trial femoral assembly for hip arthroplasty according to Claim 5, further comprising a coupling sleeve having a flange member at an outer end that engages a slot located internally within the head.

8. The trial femoral assembly for hip arthroplasty according to Claim 7, wherein the coupling sleeve is attached to the neck.

9. The trial femoral assembly for hip arthroplasty according to any one of the preceding claims, further comprising a plurality of nested, telescoping threaded elements with oppositely handed threads that extend or retract the neck.

10. The trial femoral assembly for hip arthroplasty according to Claim 9, further comprising a first nested, telescoping threaded element attached to the stem or broach, a third nested, telescoping threaded element attached to the head, and a second nested, telescoping threaded element located between the first nested, telescoping threaded element attached to the stem or broach and the third nested, telescoping threaded element attached to the head.

11. The trial femoral assembly for hip arthroplasty according to Claim 10, further comprising a threaded nut that can travel up and down the first nested, telescoping threaded element and an anti-rotation guide located between the first nested, telescoping threaded element and the third nested, telescoping threaded element to lock rotation of the first nested, telescoping threaded element and the third nested, telescoping threaded element in relationship to each other.

12. The trial femoral assembly for hip arthroplasty according to Claim 11, wherein there is at least a fifty percent overlap between the plurality of nested, telescoping threaded elements at any one time and the threaded nut has threads in the same direction as the third nested, telescoping threaded element and of an opposite direction to that of the first nested, telescoping threaded element, wherein the third nested, telescoping threaded element is not rotating relative to the first nested, telescoping threaded element so that rotation of the head does not affect an overall length of the trial femoral assembly.
